# EUROPEAN PATENT SPECIFICATION

(11) **EP 1 293 188 B1**
(45) Date of publication and mention of the grant of the patent: **15.08.2007**
(21) Application number: 01948173.8
(22) Date of filing: 21.06.2001
(51) Int. Cl.: A61F 5/01

(54) **Body support device**
Körperstützvorrichtung
Dispositif de support pour le corps

(30) Priority: 21.06.2000 RU 2000115794
(43) Date of publication of application: 19.03.2003
(73) Proprietor: Zakrytoe Aktsionernoe Obschestvo Nauchno-Proizodstvenny Tsentr Ogonek, Moscow, 127273 (RU)
(72) Inventor: AVERIANOV, Andrei Igorevich, Moscow, 125422 (RU); SEMENOVA, Xenia Alexandrovna, Moscow, 117330 (RU); CHUGUNOV, Vitaly Viktorovich, Moscow, 125015 (RU)
(74) Representative: Huntingford, David Ian
(86) International application number: PCT/RU2001/000249
(87) International publication number: WO 2002/000156

(56) References cited:
- EP-A1- 0 502 792
- WO-A-00/15307
- WO-A1-93/12739
- DE-A- 2 602 492
- FR-A1- 2 618 325
- RU-A- 2 131 232
- RU-C1- 2 054 907
- US-A- 3 868 952
- US-A- 4 422 453
- US-A- 4 969 452
- US-A- 5 154 690
- US-A- 5 860 944

## Description

### Technical Field

The present invention relates to medicine, and more particularly to a device for treating patients with sequels of the central nervous system affection and/or of injury to the locomotorium.

Reference is made herein to fasteners of the "fabric hook and loop type", namely fasteners of the type comprising a strip having a plurality of hooks on its surface that fasten a corresponding strip having a surface carrying a plurality of loops, such fasteners being sold often under the Registered Trade Mark "VELCRO".

### BACKGROUND ART

The problem of treating the diseases is urgent not only due to the presence of a great many patients suffering from such diseases but also on account of the quality of the heretofore-known methods of treatment.

In the present time various devices are known to use for restoring the locomotor functions of the various portions of human musculoskeletal system.

For instance, practically all the heretofore-known orthopedic devices (both metal-plastics and splint-leather) for treating pathologies of the lower extremities are formed as the so-called "exterior skeleton" which protects the functions of muscles and capsuloligamentous apparatus of the joints of the upper and lower extremities. US Patent No. 5,658,242 discloses a device for facilitating locomotor functions of the lower extremities, comprising a lumbar supporting element (bandage) which is connected through a resilient-elastic brace to the ankle-joint and hip-joint supporting bandages. The said bandages are interconnected through rigid braces, which have a hinge connection in their middle portion at the level of the knee joint.

This device release load from the muscles of lower extremities during walking and contributes to some restoration of locomotor functions of the legs of the patient which sustained a trauma or affection of lower extremities.

However, the use of the rigid "exterior skeleton" leads to weakening and hypotrophy of the muscles of the lower extremities.

Said known devices may be used during treatment of the musculoskeletal system of the upper extremities for treating various portions thereof, for instance, "Unloading bandage on upper extremity" (left/right)", "Elbow bandage", "Bandage on a wrist". "Splint of the first finger", "Hand holder" (cf. Catalog of orthopedic devices available from the Scientific-Production Center (SPC) "Ogoniok", Moscow, 1998, pp. 12, 13, 33 (in Russian).

However, it is difficult to use these devices simultaneously since each of them is adapted for independent use only for treatment of portionicular pathologies of upper extremities.

At the present time no self-comprised devices for restoration of musculoskeletal system of patient's upper extremities as a whole are known to the authors.

In order to eliminate sequels of injuries to and surgical interventions in the lumbar spine, one more prior-art device is widely used, which is formed as a correcting corset for the lumbar spine, said device being provided with longitudinal metallic inserts (cf. Catalog of orthopedic devices available from SPC "Ogoniok", Moscow. 1998, p. 29 (in Russian) In certain diseases said device provides fixation of the lumbar spine in a physiologically suitable position.

However, all the devices discussed above are suitable only for independent use during the treatment of a concrete patient's pathology. These devices fail to provide a possibility of their joint use in cases where such a necessity arises in view of medical indications.

One prior-art device for treating patients with disturbed posture and motor activity is known from patent of the Russian Federation No. 2,054,907 with a priority of January 31, 1992, which is formed as a suit with mutually suit with mutually complementing locomotor and dynamic elements. The locomotor elements of this suit are formed as a combination of shoulder, pelvic, knee, feet, elbow, arm and finger supports, and are interconnected by dynamic elements being formed as elastic braces, which establish a proportioned load which corrects the stature of the patient Each brace is connected to two supports by connection of one of its end to one locomotor element through a tension adjuster and a fixed connection of its other end to another locomotor element. Because of this connection, the location of the elastic braces relative to the body of the patient is provided in accordance with preliminarily selected directions, and in portionicular along the anterior, lateral and posterior surfaces of the patient's body. This position of the elastic braces may not be changed to suit a pathology of the patient. In cases where it is necessary to change a corrected posture of the patient, it is necessary to use another similar device, which is formed with account of correcting the pathology of the patient. Therefore, the device is functionally limited.

The shoulder support of this device performs the function of an upper fastening element for fixing elastic braces thereon and an upper supporting element of the suit; the pelvic support performs the function of an intermediate supporting-bracing element and produces a loading action on the spine; the feed supports are formed as a bandage on the ankle, a harness under the arch of the foot, a harness under a front portion of the foot, and performs the function of a lower fastening element for fixing thereon the elastic braces of a lower supporting element of the suit, which applies a load on the body of the patient; elbow and knee supports perform the functions of intermediate fixing supports, and each of them is formed as a bandage which tightly embraces an elbow/knee joint and severely limits the mobility of the joint.

The device provides fixation of joints in a desired position with the generation of a moment of force, which enhances flexion, extension, rotation, adduction and abduction of both the extremities and the trunk.

However, the above described arrangement of the elastic braces of the known device provides the formation of a locomotor structure only for a whole suit which provides a correcting action on the body of a patient only in the event of a predetermined correspondence to the location of the elastic braces, since the fixed character of the connection of one of the ends of each corresponding brace to the corresponding locomotor element limits the direction of setting the elastic brace and excludes provision of a universal functional device, which is adapted for treatment of patients with different dysfunctions of the locomotorium, including disorders of one of the sections of the musculoskeletal system.

Moreover, the abovedescribed device fails to provide conditions for reducing a pathological hypertonus of the greater pectoral muscles, and correspondingly, fails to reduce of a pathological synergy of the muscles of upper and lower extremities.

Moreover, the elbow locomotor element limits the flexion of a patient's arm in the elbow joint and prevents increasing a physiological angle of flexure, while the fixation of the elastic braces in the region of the radiocarpal joint excludes from the treatment process the work of a hand during the use of the known device, since the abducting brace of the first finger may not compensate for the absence of a dosed flexion and extension of the hand.

Patent of the Russian Federation No. 2,131,232, wich represents the closest prior art, discloses a device for treatment of patients with affected locomotorium, including sequels of injuries to the central nervous system. The device is essentially a suit composed of mutually complementing elements, and comprises a recliner for the upper portion of the patient's body, said recliner being formed as a supporting-setting bandage for the shoulder girdle and thoracic portion of the body, said bandage being in fact a pelt having a height not less than a distance from the seventh cervical vertebra to the lower margin of patient's scapulas and a width not less than a distance between the vertical lines that bound the medial portion of the scapulas; support-setting bandages for the upper shoulder girdle, being formed as triangular pelts corresponding to the anthropometric dimensions of the patient's upper shoulder girdles and shoulder joints; a lumbar supporting-setting bandage being formed as a belt provided with a device for mutual fixing to other supporting-setting elements; supporting-setting bandages for the lower extremities in the form of knee caps and popliteal supports, bandages for the anterior portion of each foot, bandages for each ankle joint and each calcareal region.

The supporting-setting bandage for the shoulder girdle and thoracic portion of a body reclines the muscles of the thoracic spine, performs the function of a supporting-setting element and portionicipates in establishing single flexible carcasses by connection to the bandages for the shoulder girdles. Said recliner operates as a correcting device of a reflex-loading type and is a most important setting element for forming a single flexible carcass.

The supporting-setting bandages for the upper shoulder girdles serve for securing thereto the fastening elements and forming flexible carcasses for the body and lower extremities of the patient.

The lumbar supporting-setting bandage performs the function of a supporting-setting element, to which flexible braces are secured. It portionicipates in forming a single flexible carcass for the body and lower extremities.

The supporting-setting bandages for the lower extremities are adapted for fixing thereto the correcting-rotating elements and portionicipate in forming a single flexible carcass for the body and lower extremities of the patient. The knee caps are used for correcting the position of the knee joint relative to the hip joint, while the popliteal supports are used for providing an additional correcting the foot and ankle joint The knee caps and popliteal supports are not used simultaneously.

The bandages for the anterior portion of each foot, the bandages for each ankle joint and for each calcareal region are supporting-setting elements for forming a single flexible carcass in the region of the ankle joint and foot.

All the elements of the device are interconnected through longitudinal fixing members (fastening members) which form a flexible carcass for the body and extremities of the patient, and also by elastic braces which perform the functions of correcting-rotating elements. The size and place of installation of said elastic braces may be changed to suit physiological and muscular synergies of the patient.

The device allows of weakening or temporarily eliminating the effect of a cervical symmetric tonic reflex and a labyrinth tonic reflex of the patient due to provision conditions for a more complete drawing aportion patient's humeral articulations and their fixing in such a position. This is attained due to using a supporting-setting bandage for the upper shoulder girdle, and a recliner for the thoracic portion of trunk. In addition, the herein-proposed embodiment of said components of the known device somewhat reduces, as compared with the device disclosed in Patent of the Russian Federation No. 2,054,907, a loading effect on the spine, which in turn is favorable for the patient's locomotor and ligamentous apparatus.

However, the device gives a therapeutic effect only when the suit is used as a whole. When it is necessary to produce a therapeutic action only on a particular portion of the musculoskeletal system of the patient, it is necessary to use the device as a whole, while the use of its fragment alone in the form of the supporting-setting bandage which corresponds to the injured portion of said system fails to produce a therapeutic effect since it may not guarantee a required correction without the use of the device as a whole, which makes it possible to develop a tightening force of the correcting-rotating elements which is necessary for correction.

It is also necessary to mention that all the heretofore-known devices for treating patients with sequels of a central nervous system and locomotorium affection may be considered as power-transmission chains which apply a more or less pronounced load on all portions of the spinal column without a real compensation for possible deforming effect, in view of the fact that in a majority of cases the patients have a pathology of the spinal column.

### Disclosure of the Invention

It is a primary object of the present invention to provide a universal modular device for patients with diseases of the central nervous system and/or of injury to the locomotorium, whose modules are adapted for independent use in treating an affected portion of patient's musculoskeletal system so as to provide fixing the patient's spine in a corrected position without applying any load on the spine, to restore a correct physiological attitude of patient's body both at rest and during motion, to reduce pathologic reflexes, and normalizing patient's motions, as well as developing a motion pattern close to the normal one.

The foregoing object is accomplished due to the provision of a device for treating patients suffering from diseases of the central nervous system and/or of injury to the locomotorium thereof, comprising a recliner located in an upper region of the patient's body and adapted for abducting the shoulder girdles and adducting the scapulas to the spine; means for correcting the middle region of the patient's body located in the lumbar region thereof; means for correcting the hip and shin with a possibility of fixing the hip and shin in a predetermined position; at least one means for correcting the ankle joint and toes, capable of fixing the foot relative to the ankle joint; a plurality of correcting-rotating elements and a plurality of connecting means, wherein, according to the present invention, said elements of the device is formed as separate modules which cover certain regions of the patient's body, each of said modules being adapted for independent use; said recliner being in fact a first module; said means for correcting the middle region of patient's body is essentially a second module adapted for correcting the spine without applying a vertical load thereon; at least one means for correcting the hip and shin is a third module adapted for fixing the hip and shin in a predetermined position so as to provide freedom of motion for the knee joint; and at least one means for correcting the ankle joint and toes is essentially a fourth module adapted for fixing the foot relative to the ankle joint in the frontal and sagittal planes in order to provide freedom of motion in the ankle joint; an exterior surface of each module is made from a napped material adapted for use in a fabric hook and loop type fasteners'; said correcting-rotating elements successively join together said second, third and fourth modules with a possibility of their disjoining; each correcting-rotating element is formed as a band from elastic material having a percentage elongation of from 5 to 50% so as to provide correction of the patient's motion during his/her displacement and has a number of connecting means adapted to adjust tightening of said correcting-rotating elements at each place of their connection to each of said second, third, and fourth modules, each of said connecting means having an engaging surface adapted for providing a connection of the fabric hook and loop type of fastener to the exterior surface of each of the second, third, and fourth modules at any place on said surface depending on the pathology the patent is suffering from.

It is expedient that each correcting-rotating element have a means for varying the length thereof.

It is advantageous that the recliner have a fust flexible band and a second flexible band, each of them being spatially curved in the form of a loop such that a fust strap and a second strap is formed, each embracing the respective patient's shoulder joint and having an interaction means adapted to interconnect the fust ends of said first and second flexible bands with a possibility of adjusting the spacing therebetween, said means being disposed on the patient's back, while the second ends of the first and second flexible bands are secured on the respective first and second flexible bands nearby said interaction means.

It is desirable that the device further comprise an elastic plate covering a portion of the patient's back in the region of the scapulas between the recliner and the patient's back; the exterior surface of said elastic plate is made from a napped material adapted for use in a fabric hook and loop type fastener for connection to the first and second recliner bands; the inner side of each band has an engaging surface adapted for use in the fabric hook and loop type fastener for connection to the exterior surface of said elastic plate the lower portion of which has a connecting interconnecting means to the second module.

It is advantageous that the means for correcting the middle portion of the patient's body comprise a corset having a profiled shape that provides spine correction without applying a vertical load thereon, a first portion of said corset embracing the patient's body in the lumbar region and having a means for transversely fixing said portion on the patient's body, while a second portion of the corset covers the patient's back in the region of the scapulas.

It is expedient that the means for correcting the middle portion of the patient's body comprise the following components: a corset having a profiled shape to provide spine correction without applying a vertical load thereon, said corset embracing the patient's body in the lumbar region and being provided with a means for its being fixed transversely on the patient's body and a connecting means adapted to be joined to the connecting means for said plate.

It is possible that said means for correcting the hip joint and the ankle joint comprise a first flexible bandage and a second flexible bandage, both embracing the lower extremity over and under the knee joint, respectively and being fixed there with a possibility of adjusting the spacing therebetween using a fastener provided at one end of each of said flexible bandages which are interconnected in the popliteal region with their opposite longitudinal edges.

It is useful that said means for correcting the ankle joint and toes comprise a first flexible belt embracing the ankle and having at one of its ends an interaction means adapted for fixing said flexible belt to the ankle with a possibility of adjusting the distance therebetween; a second flexible belt embracing the foot in the region of the longitudinal plantar arch thereof and having its ends attached to the first flexible belt on the opposite lateral ankle surfaces; a cross-shaped toe-plantar element having three ends spatially curved toward one another to embrace the foot in the region of the toe, said ends being interconnected through fastener, while a fourth end thereof is vacant and is disposed under the foot along the entire length thereof; provision is also made for two elastic braces which connect the respective first and second flexible belts to said toe-plantar element in the region of the toe with a possibility of adjusting the spacing therebetween.

It is desirable that the device further comprise setting-connecting means for interconnecting the correcting-rotating elements with a corresponding module, each of said means having at least one bolt loop and is made up of two layers, a first of said layers having its exterior surface made from a napped material adapted for use in a fabric hook and loop type fastener, while the exterior surface of the second layer is an engaging one adapted for use in the fabric hook and loop type fastener; each of said connecting means provided in each of the correcting-rotating elements has a napped surface adapted for use in the fabric hook and loop type fastener.

It is expedient that the device further comprise a fifth module being formed as at least one means for correcting an upper extremity adapted for fixing the shoulder and forearm in a preset position in order to provide freedom of motion for the elbow joint, the exterior surface of said module being made from a napped material adapted for use in a fabric hook and loop type fastener; provision being made for at least one correcting-rotating element (33) which connects said fifth module (E) to said first module (A) with a possibility of their being disconnected from each other, said element being formed as a band from an elastic material having a percentage elongation of from 5 to 50% and adapted to correct motion of an upper extremity during its functioning, said correcting-rotating element being provided with a number of connecting means adapted to adjust tightening of said element at the place of its connection to said first and fifth modules, and with an engaging surface adapted for use in a fabric hook and loop type fastener at any place on the exterior surfaces of said first and fifth modules depending on the pathology the patient suffers from.

It is desirable that each correcting-rotating element of the means for correcting an upper extremity be provided with a means for varying the length thereof.

It is advantageous that the means for correcting an upper extremity comprise a first spatially curved flexible band and a second spatially curved flexible band, both of them embracing the upper extremity over and under the elbow joint, respectively, and being fixed there with a possibility of adjusting the distance therebetween by means of fastener provided at one end of each of said spatially curved flexible bands, said flexible bands being interconnected by the opposite longitudinal edges thereof; there is provided a V-shaped flexible carpal element ergonomically adapted for being fixed to the palm and for abducting the thumb, and a means for fixing said flexible carpal element to the palm with a possibility of adjusting the tightening force of said element which is connected to the second flexible band through at least one elastic brace which interconnects said flexible carpal element with said second flexible band, provision being made at the ends of said elastic brace for connecting means adapted to adjust tightening of said brace at the place of its connection to said flexible carpal element and to said second flexible band.

The abovedescribed construction arrangement of the herein-proposed device provides for establishing a universal modular device whose individual modules are suitable for independent use in treating an appropriate afflicted portion of the patient's musculoskeletal system or of the central nervous system thereof.

Moreover, the device is capable of fixing the patient's spine in the corrected position without applying a vertical load thereon.

A possibility is also provided of restoring a correct physiological spatial attitude of the patient, both at rest and during motion, as well as of reducing pathologic reflexes, normalizing patient's motions, and developing a motion pattern close to the normal one.

Each of said modules may differ as to the construction arrangement from that described before and may have another construction arrangement suitable for similar purposes.

However, said objects may efficiently be accomplished due to the provision of a device for treating patients suffering from sequels of the central nervous system affection and/or of injury to the locomotorium thereof, which device comprises a recliner located in an upper region of the patient's body and adapted for abducting the shoulder girdles and adducting the scapulas to the spine; means for correcting the middle region of the patient's body located in the lumbar region thereof; at least one means for correcting the hip and shin adapted for fixing the hip and shin in a predetermined position; at least one means for correcting the ankle joint and toes, capable of fixing the foot relative to the ankle joint; a plurality of correcting-rotating clements and a plurality of connecting means, wherein, according to the present invention, the elements of the device are formed as independent modules which cover certain regions of the patient's body, each of said modules being adapted for independent use; said recliner being in fact a first module which comprises a first flexible band and a second flexible band, each being spatially curved to form a loop such that a first strap and a second strap is formed, each embracing a respective patient's shoulder joint and having an interaction means adapted to interconnect the first ends of said first and second flexible bands with a possibility of adjusting the spacing therebetween, said means being disposed on the patient's back, and the ends of said first and second flexible bands are secured on said respective first and second flexible bands nearby said interaction means; said means for correcting the middle portion of the patient's body is a second module adapted for correcting the spine without applying a vertical load thereon and comprising a corset having a profiled shape that provides spine correction without applying a vertical load thereon, a first portion of said corset embracing the patient's body in the lumbar region and having a means for transversely fixing said portion on the patient's body, while a second portion of the corset covers the patient's back in the region of the scapulas; at least one means for correcting the hip and shin is a third module adapted for fixing the hip and shin in a predetermined position so as to provide freedom of motion for the knee joint and comprising a first flexible bandage and a second flexible bandage, both embracing the lower extremity over the knee joint and under the knee joint, respectively, and fixed therein with a possibility of adjusting the spacing therebetween by means of fastener provided at one end of each of said flexible bands, said bands being interconnected by their opposite longitudinal edges, and at least one means for correcting the ankle joint and toes is a fourth module adapted for fixing the foot relative to the ankle joint in the frontal and sagittal planes with a view to providing freedom of motion in the ankle joint and comprising a first flexible belt embracing the ankle and having at one of its ends an interaction means adapted for fixing said flexible belt to the ankle with a possibility of adjusting a distance therebetween; a second flexible belt embracing the foot in the region of the longitudinal plantar arch and having its ends attached to the first flexible belt on the opposite lateral ankle surfaces; a cross-shaped toe-plantar element having three ends spatially curved toward one another to embrace the foot in the region of the toe, said ends being interconnected through fasteners, while a fourth end is vacant and is disposed under the foot along the entire length thereof; provision is also made for two elastic braces which connect the respective first and second flexible belts to said toe-plantar element in the region of the toe with a possibility of adjusting the spacing therebetween; the exterior surface of each module is made from a napped material adapted for use in the a fabric hook and loop type fastener, said correcting-rotating elements are adapted to consecutively interconnect the second, third and fourth modules with a possibility of their disconnection, and each correcting-rotating element is formed as a band from an elastic material having a percentage elongation of from 5 to 50% so as to provide correction of the patient's motion during his/her displacement and has a number of connecting means adapted to adjust tightening of said correcting-rotating elements at each place of their connection to each of said second, third, and fourth modules, each of said connecting means having an engaging surface adapted for providing a connection of the fabric hook and loop fastener to the exterior surface of said modules at any place on said surface depending on the pathology the patient is suffering from.

It is expedient that each correcting-rotating element have a means for varying the length thereof.

It is advantageous that the device further comprise a fifth module being formed as at least one means for correcting an upper extremity adapted for fixing the shoulder and forearm in a preset position and providing freedom of motion for the elbow joint the exterior surface of said module being made from a napped material adapted for use in fabric hook and loop type fastener; the means for correcting an upper extremity comprise a first spatially curved flexible band and a second spatially curved flexible band, both embracing the upper extremity over and under the elbow joint, respectively, and being fixed there with a possibility of adjusting the distance therebetween by means of fastener provided at one end of each of said spatially curved flexible bands, said bands being interconnected by the opposite longitudinal edges thereof; there is provided a V-shaped flexible carpal element ergonomically adapted for being fixed to the palm and for adducting the thumb, and a means for fixing said flexible carpal element to the palm with a possibility of adjusting the tightening force of said element which is connected to the second flexible band through at least one elastic brace which interconnects said flexible carpal element with said second flexible band, provision being made at the ends of said elastic brace for connecting means adapted to adjust tightening of said brace at the place of its connection to said flexible carpal element and to said second flexible band; there is provided at least one correcting-rotating element which connects said fifth module to said first module with a possibility of their being disconnected from each other, said element being formed as a band from an elastic material having a percentage elongation of from 5 to 50% and adapted to correct motion of an upper extremity during its functioning, said correcting-rotating element being provided with a number of connecting means adapted to adjust tightening of said element at the place of connection to said first and fifth modules, and with an engaging surface adapted for use in a fabric hook and loop type fastener at any place on the exterior surfaces of said first and fifth modules depending on the pathology the patient is suffering from.

It is desirable that each correcting-rotating element of the means for correcting an upper extremity be provided with a means for varying the length thereof.

Said objects may efficiently be accomplished due to the provision of a device for treating patients suffering from sequels of the central nervous system affection and/or of injury to the locomotorium thereof, which device comprises a recliner located in an upper region of the patient's body and adapted for abducting the shoulder girdles and adducting the scapulas to the spine; means for correcting the middle region of the patient's body located in the lumbar region thereof; at least one means for correcting the hip and shin adapted for fixing the hip and shin in a predetermined position; at least one means for correcting the ankle joint and toes, capable of fixing the foot relative to the ankle joint; a plurality of correcting-rotating elements and a plurality of connecting means, wherein, according to the present invention, said elements of the device are formed as independent modules which cover certain regions of the patient's body, each of said modules being adapted for independent use; said recliner is in fact a first module which comprises a first flexible band and a second flexible band, each being spatially curved to form a loop such that a first strap and a second strap is formed, each embracing a respective patient's shoulder joint and having an interaction means adapted to interconnect the first ends of said first and second flexible bands with a possibility of adjusting the spacing therebetween, said means being disposed on the patient's back, and the ends of said first and second flexible bands are secured on said respective first and second flexible bands nearby said interaction means; said means for correcting the middle portion of the patient's body is a second module adapted for correcting the spine without applying a vertical load thereon and comprising a corset having a profiled shape that provides spine correction without applying a vertical load thereon, a first portion of said corset embracing the patient's body in the lumbar region and having a means for transversely fixing said portion on the patient's body, while a second portion of the corset covers the patient's back in the region of the scapulas; at least one means for correcting the hip and shin is a third module adapted for fixing the hip and shin in a predetermined position so as to provide freedom of motion for the knee joint and comprising a first flexible bandage and a second flexible bandage, both embracing the lower extremity over the knee joint and under the knee joint, respectively, and fixed therein with a possibility of adjusting the spacing therebetween by means of fastener provided at one end of each of said flexible bands, said bands being interconnected by their opposite longitudinal edges, and at least one means for correcting the ankle joint and toes is a fourth module adapted for fixing the foot relative to the ankle joint in the frontal and sagittal planes with a view to providing freedom of motion in the ankle joint and comprising a first flexible belt embracing the ankle and having at one of its ends an interaction means adapted for fixing said flexible belt to the ankle with a possibility of adjusting a distance therebetween; a second flexible belt embracing the foot in the region of the longitudinal plantar arch and having its ends attached to the first flexible belt on the opposite lateral ankle surfaces; a cross-shaped toe-plantar element having three ends spatially curved toward one another to embrace the foot in the region of the toe, said ends being interconnected through fastener, while a fourth end is vacant and is disposed under the foot along the entire length thereof; provision is also made for two elastic braces which connect the respective first and second flexible belts to said toe-plantar element in the region of the toe with a possibility of adjusting the spacing therebetween; the device further comprises a fifth module being formed as at least one means for correcting an upper extremity adapted for fixing the shoulder and forearm in a preset position and providing freedom of motion for the elbow joint, said means for correcting an upper extremity comprise a first spatially curved flexible band and a second spatially curved flexible band, both embracing the upper extremity over and under the elbow joint, respectively, and being fixed there with a possibility of adjusting the distance therebetween by means of fastener provided at one end of each of said spatially curved flexible bands, said bands being interconnected by the opposite longitudinal edges thereof; there is provided a V-shaped flexible carpal element ergonomically adapted for being fixed to the palm and for adducting the thumb, and a means for fixing said flexible carpal element to the palm with a possibility of adjusting the tightening force of said element which is connected to the second flexible band through at least one elastic brace which interconnects said flexible carpal element with said second flexible band, provision being made at the ends of said elastic brace for connecting means adapted to adjust tightening of said brace at the place of its connection to said flexible carpal element and to said second flexible band; the exterior surface of each module is made from a napped material adapted for use in a fabric hook and loop type fastener, said correcting-rotating elements are adapted to consecutively interconnect the second, third and fourth modules with a possibility of their disconnection, and each correcting-rotating element is formed as a band from an elastic material having a percentage elongation of from 5 to 50% so as to provide correction of the patient's motion during his/her displacement and has a number of connecting means adapted to adjust tightening of said correcting-rotating elements at each place of their connection to each of said modules, each of said connecting means having an engaging surface adapted for providing a connection of the fabric hook and loop type fastener to the exterior surface of said modules at any place on said surface depending on the pathology the patient is suffering from.

It is expedient that each correcting-rotating element have a means for varying the length thereof.

The present invention provides condition for fixing the patient's spine in a corrected position, and also for its unloading and traction during diagnosing in the patient with paralytic scoliosis, Sheuermann-Mau disease, traumatic injury to the vertebral bodies, in cases of a typical "round" back, an infantile cerebral paralysis, osteochondrosis of the spine, and other similar diseases.

The proposed device, due to the mechanical traction correction without applying a load on the spine, contributes to removal of action of tonic reflexes due to abducting the shoulder girdles and their stable fixation in that position. The proposed invention provides a reflex reduction of a pathological tonus of the pectoral muscles and muscles of the pelvic and shoulder girdles, reflex reduction of a tonus of lower extremities and therefore provides a possibility of positioning and fixing of maximum physiological positions of upper and lower extremities in the shoulder, elbow, radiocarpal, hip, knee, and ankle joints and joints of foot at rest and during motion. Moreover, the proposed invention provides normalization of muscular tonus and increase of muscle strength and sustaining power which may be qualified as a result of training-simulator function of the proposed device.

The proposed invention does not provide a single flexible carcass for the patient's body and therefore eliminates all abovementioned negative results when it is provided. At the same time each module of the proposed invention performs its function to accomplish the set objects, for instance the recliner formed in accordance with the present invention is used for forming a correct posture, the correcting corset which is formed in accordance with the present invention provides correcting of the spine without applying a load thereon, a complex bandage for the hip and shin and a correcting device for the ankle joint and toes in accordance with the present invention and make possible the use of other known correcting devices simultaneously with the proposed device.

In order to extend the functional capabilities of the proposed invention, in paricular for treating patients who sustained, e.g., cerebro-vascular accident or infantile cerebral paralysis involving spastic muscular tonus in the upper extremities, the device has the fifth module which is connected to the first module by at least one correcting-rotating element.

The construction arrangement of the fifth module makes possible simultaneously correcting the pathology of an entire upper extremity, at the same time preserving the freedom of motion for the elbow joint.

In order to extend functional capabilities of the proposed device and in particular for treating, e.g., patients who sustained craniocerebral injury or trauma of the various portions of the spine, the proposed device may have a means adapted for being disposed in the region of the sternum, and/or a means adapted for being arranged in the region of the pubis, and/or a means being formed as a device for correcting the cervical spine. Provision of a means for varying the length of each correcting-rotating element makes it possible to use the same proposed device for different age groups of patients, and also to increase or reduce a load during the use of the device.

The recliner band may be of various width, including the width sufficient for forming a vest.

In accordance with the present invention, the exterior surface of the structural components of the device is made from a material which corresponds to a first surface of a fabric hook and loop type fastener, and each fastening element has the surface which is in fact the mating surface of the fabric hook and loop type fastener.

Said construction solution adds to versatility of the proposed device and makes possible its use for treatment of a great many various pathologies; moreover, the device makes it possible to change the position of some modules and their elements with respect to one another in the course of treatment without removing the device depending on the disease coursing and presence of a curative effect whereby the same device may be used in the course of treatment without making resort to any other similar devices which extends considerably the functional capabilities of the proposed device.

The proposed device may be used together with other heretofore-known orthopedic facilities adapted to correct the position of, e.g., toes or fingers of arms, or neck, or head.

The use of the proposed invention makes possible a complex correction consisting in a combination of a reduction in the load applied to the spine and developing proportioned correcting loads applied to the upper and lower extremities.

### Brief Description of the Drawings

The present invention will be best understood from the following description of specific embodiments thereof when read in connection with the accompanying drawings, wherein:
FIG. 1 is an isomeric view showing a proposed device in accordance with a first embodiment;
FIG. 2 is an isometric view showing the proposed device, in accordance with a second embodiment;
FIG. 3 is an isometric view showing the proposed device, in accordance with a third embodiment;
FIG. 4 is an isometric view showing the proposed device, in accordance with a fourth embodiment;
FIG. 5 is an isometric view showing the proposed device for treatment of patients with dysfunction of the lower extremities, in accordance with the present invention;
Fig. 6 is an isometric view showing a proposed device for treatment of patients with dysfunction of the upper extremities, in accordance with the present invention;
Fig. 7 is an isometric view showing a proposed device for treatment of patients with dysfunction of the muscles of the shoulder girdle, in accordance with the present invention;
Fig. 8 is an isometric view showing variants of fixation of correcting-rotating elements to an exterior surface of any module, in accordance with the present invention; and
Fig. 9 is an isometric view showing a correcting-rotating element having a means for varying the length thereof, in accordance with the present invention.

### Best Method of Carrying Out the Invention

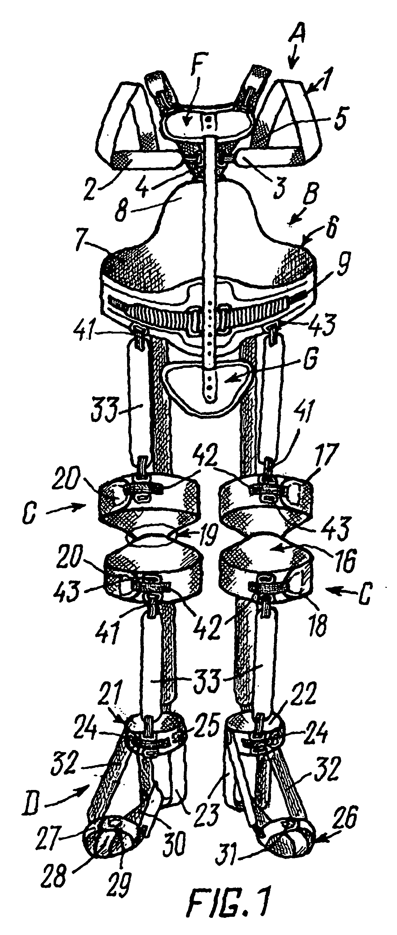

The proposed device for treatment of patients suffering from diseases of the central nervous system and/or of injury to the locomotorium in accordance with the present invention is comprised of individual modules A, B, C, D, E. (FIGS. 1. 2, 3, 4) and adapted for individual (autonomous) use, and do not provide establishing a single flexible carcass for the patient's body (the patient is not shown in the drawing). In the various embodiments of the present invention use may be made of various combinations of said modules depending on the pathology the patient suffers from. Furthermore, in the course of treatment with the use of the proposed invention any change in the mutual position of the modules or replacement of any module may be carried out.

The first module A performs reclining of the patient's shoulder girdle, otherwise speaking for forming a correct posture. It comprises a recliner 1 shown in Figs. 1, 2, 3, for an upper region of the patient's body, which comprises a first flexible band 2 and a second flexible band 2, each having a first end 3 connected by fastening elements 4 with the possibility of adjusting the distance therebetween and located on the patient's back. Used as the fastening elements 4 may be any heretofore-known fastening elements adapted for similar purposes, for instance, a latch. Each of the flexible bands 2 is formed as a spatially curved band in the form of a loop, and the second ends 5 of each flexible band 2 are fixedly connected to the band 2 nearby the fastening elements 4 so as to form the first and second straps each embracing the respective shoulder joint of the patient's body, in use.

Each flexible band 2 of the recliner 1 may be wide enough for forming a vest.

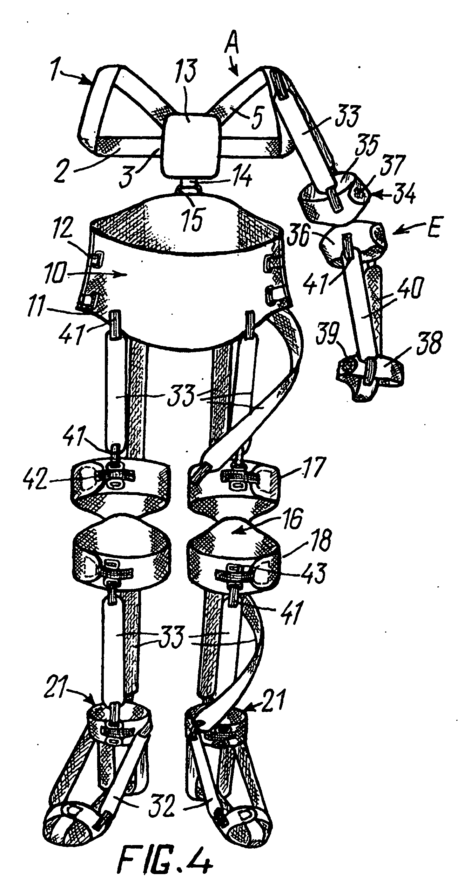

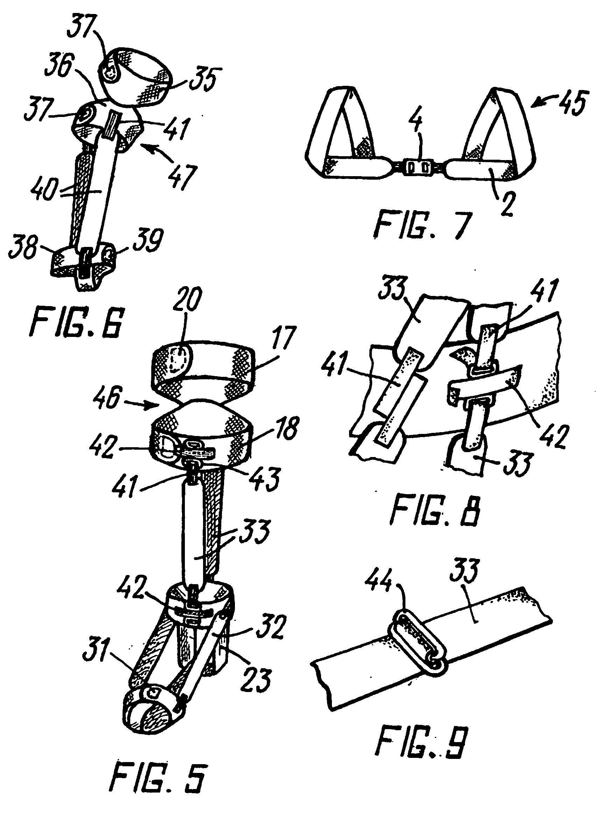

The second module B is formed as a means for correcting the middle region of the patient's body and is located, in use, in the lumbar region and adapted for correcting the spine without applying a vertical load thereon. The proposed means for correcting the middle region of the patient's body is formed as a corset 6 which has an intricately profiled shape providing correcting the spine without applying a load thereon. The corset 6 has a first portion 7 which embraces the patient's body in the lumbar region, and a second portion 8 which covers the patient's back in the zone of the shoulder blades thereof. The portions 7 and 8 comprise a single piece. The first portion 7 has means 9 for its being fixed transversely on the patient's body. In another embodiment of the invention, the proposed means for correcting the middle region of the patient's body may have only one portion 10 shown in Fig. 4, which comprises a corset 11 having an intricately profiled shape which provides correction of the spine without applying a vertical load thereon load. It embraces the patient's body in the lumbar region and has means 12 for it to be fixed transversely on the patient's body.

The device may further comprise a flexible plate 13 (Fig. 4) which covers said portion of the patient's back in the zone of the shoulder blades, and is located between the recliner 1 and the patient's back. The exterior surface of the plate 13 is composed of a napped material adapted for use as part of a hook and loop type fastener connection provided for interconnecting the first and second straps of the recliner 1, with an engaging surface of the inner side of each of them being provided for the fabric hook and loop fastener of the exterior surface of the plate 13. In the lower portion of the flexible plate 13 there is connecting means 14 for connection to the second module B which must have a connecting means 15 connectable to the connecting means 14 of the flexible plate 13.

The corset may have any other heretofore-known construction for correcting the spine without applying a vertical load thereon, for instance, the Becker's corset.

The proposed device, depending on the pathology the patient suffers from, may have a means F to be located in the region of the sternum, and/or a means G for location in the region of the pubis, and/or a means which is formed as a device for correcting the cervical spine (not shown in the drawing), or any other means complementing the proposed device.

The third module C is formed as at least one means 16 for correcting the patient's hip and shin, said means being adapted for fixing the hip and shin in a predetermined position so as to provide freedom of motion for the knee joint. When the patient have a pathology of both lower extremities, two such modules should be provided.

The means 16 for correcting the hip and shin of said lower extremity of said patient's body comprises a first flexible bandage 17 which embraces said lower extremity above said knee joint thereof and has a first end, a second end and a longitudinal edge having a middle portion; a second flexible 18 which embraces the lower extremity under the knee joint and having a first end, a second end and a longitudinal edge located opposite to the longitudinal edge of the first flexible bandage 17 and having a middle portion connected to the middle portion of the longitudinal edge of the first flexible bandage 17; a place 19 of connection of said middle portions of said opposite longitudinal edges of said first and second flexible bandage 17, 18 located in the popliteal region of the lower extremities; a first interaction means 20 (fastening element) for interconnecting said first end and said second end of the first flexible bandage 17 for its being fixed above said knee joint with a possibility of adjusting a distance between said ends; a second interaction means 20 (fastening element) adapted to interconnect said first end and said second end of said second flexible bandage 18 for its being fixed under said knee joint with a possibility of adjusting a distance between said ends.

The fourth module D is formed as at least one means 21 for correcting the ankle joint and toes of the patient's body, said means being adapted to fix the foot relative to the ankle joint in the frontal and sagittal planes so as to provide freedom of motion for the ankle joint. When the patient has pathology of both lower extremities two modules D should be provided

The means 21 for correcting the ankle joint and toes comprises a first flexible belt 22 which embraces the ankle and a second flexible belt 23 which embraces the foot in the zone of its longitudinal arch. The first flexible belt 22 has an interaction means 24 which interconnects the ends of the first flexible belt 22 for its being fixed on the ankle with the possibility of adjusting the distance between said ends. The ends of the second flexible belt 23 are secured by fastening elements 25 on the first flexible belt 22 on the opposite lateral surfaces of the ankle. The means 21 further comprises a cross-shaped toe-sole element 26 having first, second, third and fourth ends 27, 28, 29, 30, respectively. Fastening elements 31 are provided at each of the first, second and third ends 27, 28. 29. The first, second and third ends 27, 28, 29, respectively, are spatially curved toward one another and embrace the foot in the region of the toe and are interconnected by the fastening elements 31. The fourth end 30 is vacant and is disposed under said foot along its whole length including the heel. Two elastic braces 32 are also provided, each connecting the respective flexible belts 22, 23 to the toe-sole element 26 in the region of the toe with a possibility of adjusting the distance therebetween.

The interaction means 20, 24 and the fastening elements 25, 31 may have any heretofore-known construction adapted for similar purposes.

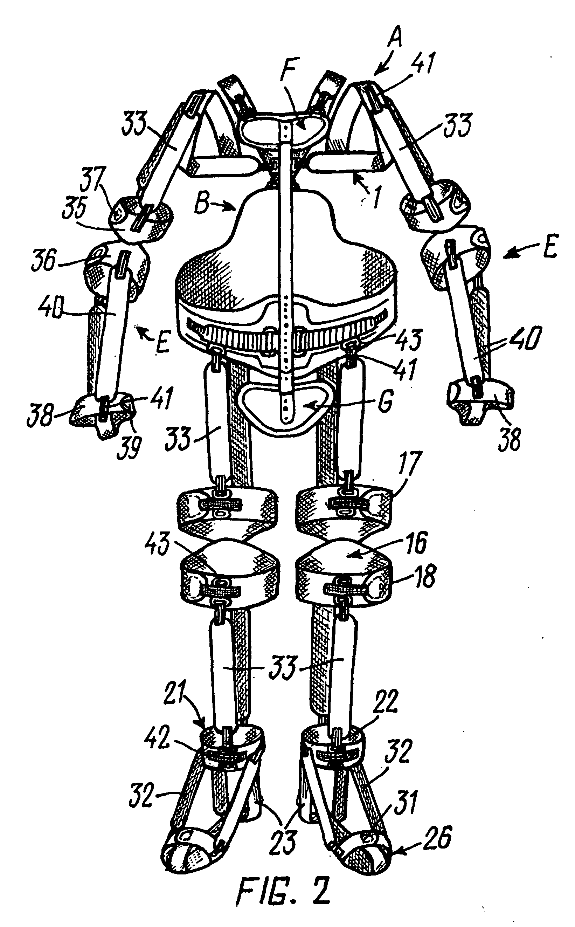

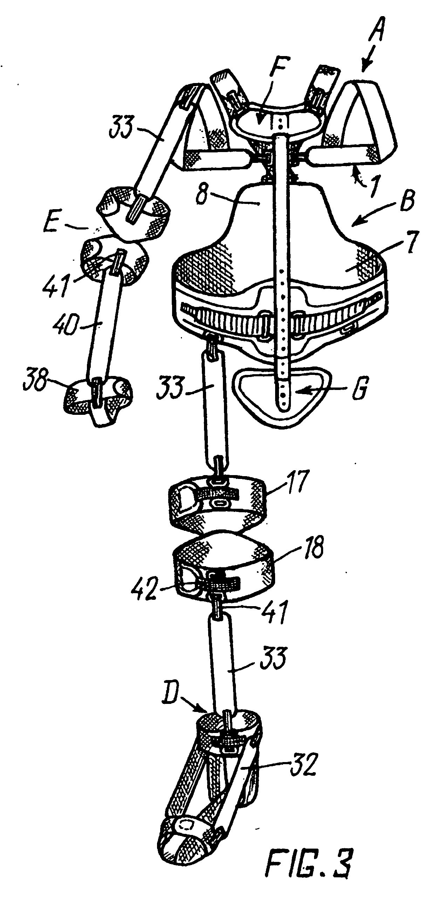

In addition, the proposed device further comprises a plurality of correcting-rotating elements 33 which consecutively connect the second, third and fourth modules with a possibility of their disconnection. The number of the correcting-rotating elements 33 depends on the pathology the patient suffers from. For instance, FIGS. 1 and 2 illustrate the proposed devices, each having four correcting-rotating elements 33 provided for the interconnection of the second and third modules. FIG. 3 shows the proposed device having single correcting-rotating element 33 for interconnecting the second and third modules. FIG. 4 shows the proposed device provided with five correcting-rotating elements 33 for interconnecting the second and third modules.

In addition, in cases of an appropriate pathology of the patient, the proposed device may comprise at least one means 34 for correcting the upper extremity of said patient's body which performs the function of a fifth module (E) adapted for fixing the shoulder and forearm in a predetermined position so as to provide freedom of motion for the elbow joint. When the patient has a pathology of both upper extremities, two means 34 should be provided.

A means 34 for correcting the upper extremity comprises a first spatially curved flexible band 35 which embraces the upper extremity above its elbow joint and have a first end, a second end and a longitudinal edge having a middle portion; a second spatially curved flexible strap 36 which embraces the upper extremity under the elbow joint and having a first end, a second end and a longitudinal edge located opposite to the longitudinal edge of the first flexible band 35 and having a middle portion connected to the middle portion of the longitudinal edge of the first flexible band 35; the place of interconnection connection between the middle portions of the opposite longitudinal edges of the first and second flexible bands 35, 36 and located in subcubital region of said upper extremity; a first interaction means 37 interconnecting the first end and the second end of the first flexible band 35 under the elbow joint with a possibility of adjusting a distance between the end; a second interaction 37 connecting the first end and second ends of the second flexible band 36 for fixing the second flexible band 36 under the elbow joint with a possibility of adjusting the distance between said ends; a carpal flexible element 38 which is ergonomically adapted for fixing on the palm and deflecting of a thumb of the upper extremity and having a V-shaped, a first and a second end each provided with a means 39 for fixing on the palm of the upper extremity with the possibility of adjusting the tightening force of the carpal flexible element 38, at least one elastic brace 40 formed similarly to the correcting-rotating elements 33 and connecting the carpal flexible element 38 to the second flexible band 36 having a first end and a second end, a means 41 for connection provided on each of the first and second ends of the elastic brace 40 for adjusting the tightening of the elastic brace 22 at the place of its connection to the carpal flexible element 28 and to the second flexible band 26.

The fifth module E is disjoinably connected to the first module A by the correcting-rotating elements 33 similar to those mentioned before. The number of the correcting-rotating elements 33 depends on the pathology the patient suffers from. For instance, FIG. 2 shows the proposed device, wherein there are provided two correcting-rotating elements 33 adapted for interconnecting each of the fifth and first module FIG. 3 shows the proposed device provided with one correcting-rotating element 33 for interconnecting the fifth and fourth modules, and FIG. 4 shows the proposed device with two correcting-rotating elements 33 for connecting the fifth and first modules.

The exterior surface of each of the modules is made from a napped material adapted for use in a fabric hook and loop type fastener.

Each of the correcting-rotating elements 33 and each of the elastic braces 40 is formed as a band of elastic material having a percentage elongation of from 5 to 50% and provides correction of the patient's motion during his/her displacement and/or functioning.

Each of the correcting-rotating elements 33 and each of the elastic braces 40 is made from a material having a percentage elongation providing a correction which corresponds to the pathology the patient suffers from, for instance, from the fabric "Tricor" (Registered Trade Mark) or "Neoprene".

Each of the correcting-rotating elements 33 has the connecting means 41 adapted for adjusting the tightening of the correcting-rotating element 33 at each place of their connection to each of the modules. Each interconnecting means 41 has an engaging surface for providing a connection of the fabric hook and loop type of fastener at any place on the exterior surface of each of the modules, depending on the pathology the patient suffers from.

Said correcting-rotating element 33 may connect the modules using the means 41 immediately as described hereinabove. However, such connection may be performed by the setting-connecting means 42, each them being made up of two layers held to each other. The exterior surface of the first layer is made of a napped material adapted for use in a fabric hook and loop type fastener, the exterior surface of the second layer is an engaging one adapted for use in the fabric hook and loop type fastener. Each of the setting-connecting means 42 has at least one strap 43. Each of the connecting means 41 provided on each of the correcting-rotating elements 33 must have a napped surface adapted for use in a fabric hook and loop type fastener.

Each of the correcting-rotating element 33 may have a means 44 for varying the length thereof. The means 44 shown in Fig. 9 may comprise, e.g., a retainer made up of two oval elements. Alternatively, it may have another heretofore-known construction adapted for similar purposes.

As it has been stated hereinabove, each of the modules A, B, C, D, E, of the proposed device, depending on the pathology the patient suffers from, may be used independently of the other modules. For instance, the first module A may be used as an independent device 45 (FIG. 7) for treating patients with muscular dysfunction of the shoulder girdle.

The third and fourth modules C, D, may be used as an independent device 46 shown (FIG. 5) for treating patients with dysfunction of the lower extremities.

The fifth module E may be used as an independent device 47 shown (FIG. 6) for treating patients with dysfunction of the upper extremities.

The proposed device functions as follows.

Considered hereinbelow is an embodiment when the pathology the patient suffers from requires the use of the proposed device as a whole, that is, use of the proposed device comprising the first, second and third, fourth and fifth modules.

First the first and second modules A, B are put on the patient, then the third, fourth and fifth modules C, D, and E. The second module B in this case is a base for setting of power chains for the lower extremities. Depending on the pathology that the patient suffers from and on medical indications associated therewith, the number and length of the correcting-rotating elements 33 are selected for interconnecting the corresponding modules. Then the third and fourth modules C, D are interconnected with the second module B, using the correction-rotating element 33, while the fifth module E is interconnected with the first module A so as to establish the proportioned power chains on the lower and upper extremities. The number and length of the correcting-rotating elements 33 which is used for interconnecting the corresponding modules is selected depending on the size of the patient and the pathology he/she is suffering from. Using the connecting means 41 all the modules are connected in accordance with the selected scheme.

Depending on the medical indications, used simultaneously with the proposed device may also be some other heretofore-known orthopedic devices, such as a variety of head holders, e.g., soft head holders, analogs to the Schanz's collar, made from a variety of materials such as staff, inflatable, and other constructions. In addition, it is possible to use various fixing devices, for instance, with orthopedic sole, shoe and orthopedic facility which are fixed on the proposed device by their fastening elements. Then the dynamic tests of the patient in the assembled device are conducted, and, if necessary, correction of the power chains is performed. In course of the use of the proposed device whenever it becomes necessary, the correcting-rotating elements may be corrected for length and also for position of their fixation to the exterior surface of the corresponding module.

### Example 1

### Male patient C.B., 32

Diagnosis: Compression breaking Th 12-L2 of vertebra with lesion of the bone marrow. Low-progressive paresis of the lower extremities. During examination of the patient, a hypotrophy of the muscles of lower extremities was found, with reflexes being weak. In the recumbent position, equino-varus position of the feet was observed. In a vertical position the feet are placed in a flat-valgus pattern. Knee joints are not closed with the quadriceps muscles. The function of the hip muscles is considerably weakened. The function of pelvic organs is practically preserved. Pronounced phenomena of post-traumatic osteochondrosis was diagnosed. Previously treatment in accordance with the known methods were performed without pronounced effect.

The pathology (the presence of post-traumatic syndrome) prevents the use of the device in accordance with Patent of the Russian Federation No. 2,054,907, since this device performs an action on the whole locomotorium of the patient which is not advisable in this case.

The patient performed a treatment course during one month with the use of the proposed device including first, second, third and fourth modules.

A session of each treatment was 60 minutes and included treatment with the device during 20 minutes, a complex of treating physical imposition of standing, sitting, lying during 30 minutes.

25 sessions in a five-day cycle were performed with breaks for one day. As a result of the treatment, an increase of physical perseverance was observed, as well as a muscle force of the lower extremities, a reduction of evening tiredness and pains in the back, considerable improvement of moving and static functions.

### Example 2

### Male patient A.B., 12

Diagnosis was established at the age of one year, being one of infantile cerebral paralysis, spastic diplegia. Intellect is retained. Pronounced impairment of the posture of the "round-back" type is noted with the kyphotic apex at the height of Th₈- Th₁₀.

Earlier there were performed medication, physiotherapeutic and orthopedic treatments in accordance with the known methods. The treatment produced but a transient effect with the subsequent recurrence with an ever increasing degree. It was recommended to the patient to use the proposed device comprising the first, second, third and fourth modules.

Each treatment session was 40 minutes and comprises walking in the device during 10 minutes, a complex of exercise treatment in the standing, sitting, and recumbent positions during 20 minutes. 20 sessions of five-day cycles were performed with a break of two days. As a result of the treatment an improvement in the motion of the static function of patient and increase of its physical endurance was observed.

### Example 3

### Male patient I.B., 52.

Diagnosis: Acute disorder of the cerebral circulation, left-side hemiparesis, disseminated osteochondrosis of the spine, herniated discs of L3, L4, L5. The patient was treated in accordance with the known methods without a significant and clinical improvement.

Use of the device of Patent of the Russian Federation No. 2,054,907 was impossible because of disseminated osteochondrosis complicated by the presence of radicular symptoms.

The proposed device was recommended to the patient, comprising the first, second, third, fourth and fifth modules. The treatment session was 45 minutes and included walking in the device during 15 minutes, and a complex of exercise treatment in the standing, sitting, and recumbent positions during 20 minutes.

The treatment sessions were performed under monitoring of arterial blood pressure. 20 sessions of five-day cycles were performed with a two-day break therebetween.

As a result of the treatment a reduction of pathological tonus in the greater pectoral muscle was observed, which made possible an increase in the scope of motions in the upper extremities, an increase in the tonus of the deltoid muscle, and elimination of subluxation symptoms. In the lumbar region a tendency toward reducing antalgic scoliosis and subjective improvement in the radicular symptoms was observed.

It will be understood that each of the elements described above, or two or more together, may also find a useful application in other types of constructions differing from the types described above.

While the invention has been illustrated and described as embodied in device for patient's suffering from diseases of the central nervous system and locomotorium affection of body, it is not intended to be limited to the details shown, since various modifications and structural changes may be made without departing in any way from the scope of the present invention as defined by the appended claims.

Without further analysis, the foregoing will so fully reveal the gist of the present invention that others may, by applying current knowledge, readily adapt it for various applications without omitting features that, from the standpoint of prior art, fairly constitute essential characteristics of the generic or specific aspects of this invention.

### Industrial Applicability

The present can find application in neurology, neurosurgery, traumatology, orthopedics, and cardiology, in particular for treating patients suffering from infantile cerebral paralysis, injuring to the spine column probably complicated by lesion of the spinal cord, also patients with osteochondrodystrophy, scoliosis, kyphoscoliosissequals or with sequels of craniocerebral injuries, as well as for rehabilitation of patients suffering from cardiovascular diseases.

In addition, the proposed device can be made use of for correcting the patient's posture, as well as a variety of training simulators for sports exercises.

## Claims

1. A device for treating patients suffering from diseases of the central nervous system and/or of injury to the locomotorium thereof, comprising a recliner (1) for location at an upper region of the patient's body and adapted for abducting the shoulder girdles and adducting the scapulas to the spine; means for correcting the middle region of the patient's body located in the lumbar region thereof; at least one means (16) for correcting the hip and shin with a possibility of fixing the hip and shin in a predetermined position; at least one means (21) for correcting the ankle joint and toes, capable of fixing the foot relative to the ankle joint; a plurality of correcting-rotating elements (33) and a plurality of connecting means (41), wherein said elements of the device are formed as a plurality of separate modules (A, B, C, D) which cover certain regions of the patient's body, each of said modules being adapted for independent use; said recliner (1) being in fact a first module (A); said means for correcting the middle region of patient's body is essentially a second module (B) adapted for correcting the spine without applying a vertical load thereon; at least one means for correcting the hip and shin is a third module (C) adapted for fixing the hip and shin in a predetermined position so as to provide freedom of motion for the knee joint; and at least one means for correcting the ankle joint and toes is essentially a fourth module (D) adapted for fixing the foot relative to the ankle joint in the frontal and sagittal planes in order to provide freedom of motion in the ankle joint; an exterior surface of each module is made from a napped material adapted for use in fabric hook and loop type fasteners; said correcting-rotating elements (33) successively join together said second, third and fourth modules (B, C, D) with a possibility of their disjoining; each correcting-rotating element (33) is formed as a band from elastic material having a percentage elongation of from 5 to 50% so as to provide correction of the patient's motion during his/her displacement and has a number of connecting means (41) adapted to adjust tightening of said correcting-rotating elements (33) at each place of their connection to each of said second, third, and fourth modules (B, C, D), each of said connecting means having an engaging surface adapted for providing a connection using a fabric hook and loop type fastener at the exterior surface of each of the second, third, and fourth modules at any place on said surface depending on the pathology the patient is suffering from.

2. A device as defined in claim 1, wherein each correcting-rotating element (33) has a means (44) for varying the length thereof.

3. A device as defined in claim 1, wherein the recliner (1) has a first flexible band (2) and a second flexible band (2), each of them being spatially curved in the form of a loop such that a first strap and a second strap is formed, each for embracing the respective shoulder joint of the patient and having an interaction means (4) adapted to interconnect the first ends (3) of said first and second flexible bands (2) with a possibility of adjusting the spacing therebetween, said means being disposed on the patient's back, while the second ends (5) of the first and second flexible bands (2) are secured on the respective first and second flexible bands (2) near to said interaction means (4).

4. A device as defined in claim 3, further comprising an elastic plate (13) for covering a portion of the patient's back in the region of the scapulas between the recliner (1) and the patient's back; the exterior surface of said elastic plate (13) is made from a napped material adapted for use in a fabric hook and loop type fastener for connection to the first and second bands (2) of the recliner (1); the inner side of each band has an engaging surface adapted for use in the fabric hook and loop type fastener for connection to the exterior surface of said elastic plate (13) the lower portion of which has a connecting means (14) for connection to the second module (B).

5. A device as defined in claim 1, wherein the means for correcting the middle portion of the patient's body comprise a corset (6) having a profiled shape that provides spine correction without applying a vertical load thereon, a first portion (7) of said corset (6) is adapted to embrace the patient's body in the lumbar region and has a means (9) for transversely fixing said portion on the patient's body, while a second portion (8) of the corset (6) is adapted to cover the patient's back in the region of the scapulas.

6. A device as defined in claim 4, wherein the means for correcting the middle portion of the patient's body comprises a corset having a profiled shape to provide spine correction without applying a. vertical load thereon, said corset embracing the patient's body in the lumbar region and being provided with a means (12) for it to be fixed transversely on the patient's body, and a connecting means (15) adapted to be joined to the connecting means (14) of said elastic plate (13).

7. A device as defined in claim 1, wherein the means (16) for correcting the hip joint and the ankle joint comprises a first flexible bandage (17) and a second flexible bandage (18), both being adapted to embrace the patient's lower extremity over and under the knee joint, respectively, and being fixed there with a possibility of adjusting the spacing therebetween using fasteners (20) provided at one end of each of said flexible bandages (17, 18) which are interconnected in the popliteal region with the opposite longitudinal edges thereof.

8. A device as defined in claim 1, wherein the means (21) for correcting the ankle joint and toes comprises a first flexible belt (22) embracing the ankle and having at one of its ends an interaction means (24) adapted for fixing said flexible belt (22) to the ankle with a possibility of adjusting the distance therebetween; a second flexible belt (23) adapted to embrace the foot in the region of the longitudinal plantar arch thereof and having its ends attached to the first flexible belt (22) on the opposite lateral ankle surfaces; a cross-shaped toe-plantar element (26) having three ends (27, 28, 29) spatially curved toward one another to embrace the foot in the region of the toe, said ends being interconnected through fasteners (31), while a fourth end (30) thereof is vacant and is adapted to be disposed beneath the foot along the entire length thereof; provision is also made for two elastic braces (32) which connect the respective first and second flexible belts (22, 23) to said toe-plantar element (26) in the region of the toe with a possibility of adjusting the spacing therebetween.

9. A device as defined in claim 1, further comprising setting-connecting means (42) for interconnecting the correcting-rotating elements (33) with a corresponding module; each of said means having at least one bolt loop (43) and is made up of two layers, a first of said layers having its exterior surface made from a napped material adapted for use in a fabric hook and loop type fastener, while the exterior surface of the second layer is an engaging one adapted for use in the fabric hook and loop type fastener; each of said connecting means provided in each of the correcting-rotating elements (33) has a napped surface adapted for use in the fabric hook and loop type fastener.

10. A device as defined in claim 1, further comprising a fifth module (E) being formed as at least one means (34) for correcting an upper extremity, adapted for fixing the patient's shoulder and forearm in a preset position in order to provide freedom of motion for the elbow joint, the exterior surface of said module being made from a napped material adapted for use in a fabric hook and loop type fastener; provision being made for at least one correcting-rotating element (33) which connects said fifth module (E) to said first module (A) with a possibility of their being disconnected from each other, said element being formed as a band from an elastic material having a percentage elongation of from 5 to 50% and adapted to correct motion of an upper extremity during its functioning, said correcting-rotating element (33) being provided with a number of connecting means (41) adapted to adjust tightening of said element at the place of its connection to said first module (A) and said fifth module (E), and with an engaging surface adapted for use in the fabric hook and loop type fastener at any place on the exterior surfaces of said first module (A) and said fifth module (E) depending on the pathology the patient suffers from.

11. A device as defined in claim 10, wherein the correcting-rotating element (33) of the means (34) for correcting an upper extremity is provided with a means (44) for varying the length thereof.

12. A device as defined in claim 10 or 11, wherein the means (34) for correcting an upper extremity comprises a first spatially curved flexible band (35) and a second spatially curved flexible band (36), both of them adapted to embrace the upper extremity over and under the elbow joint, respectively, and being fixed there with a possibility of adjusting the distance therebetween by means of fasteners provided at one end of each of said spatially curved flexible bands (35, 36), said flexible bands (35, 36) being interconnected in the subcubital region by the opposite longitudinal edges thereof; there is also provided a flexible V-shaped carpal element (38) ergonomically adapted for being fixed to the patient's palm and for abducting the thumb, and a means (39) for fixing said flexible carpal element (38) to the patient's hand with a possibility of adjusting the tightening force of said flexible carpal element (38) which is connected to the second flexible band (36) through at least one elastic brace (40) which interconnects said flexible carpal element (38) with said second flexible band (36), provision being made at the ends of said brace (40) for a number of connecting means (41) adapted to adjust tightening of said brace (40) at the place of its connection to said flexible carpal element (38) and to said second flexible band (36).

## Patentansprüche

1. Vorrichtung zur Behandlung von Patienten, die an Erkrankungen des zentralen Nervensystems und/oder Verletzungen des zugehörigen Bewegungsapparates leiden, mit einer Reklinationsorthese (1), die an einer oberen Körperregion des Patienten anzubringen und geeignet ist, den Schultergürtel zu abduzieren und die Schulterblätter an die Wirbelsäule heranzuführen; Mittel zur Korrektur des Rumpfs des Patienten, das sich im Lendenwirbelsäulenbereich desselben befindet; mindestens ein Mittel (16) zur Korrektur von Hüfte und Schienbein, wobei die Möglichkeit besteht, Hüfte und Schienbein in einer vorher eingestellten Position zu stabilisieren; mindestens ein Mittel (21) zur Korrektur des Fußgelenks und der Zehen, wobei der Fuß in Bezug auf das Fußgelenk stabilisiert werden kann; eine Vielzahl von drehbaren Korrekturelementen (33) und eine Vielzahl von Verbindungsmitteln (41), wobei die genannten Teile der Vorrichtung mehrere separate Module (A, B, C, D) bilden, die bestimmte Körperpartien des Patienten abdecken und wobei jedes der genannten Module für eine von den anderen unabhängige Benutzung geeignet ist; die Reklinationsorthese (1) stellt nämlich ein erstes Modul (A) dar, das Mittel zur Korrektur des Rumpfs des Patienten stellt im Wesentlichen ein zweites Modul (B) dar, das geeignet ist, die Wirbelsäule zu korrigieren, ohne sie dabei in senkrechter Richtung zu belasten; mindestens ein Mittel zur Korrektur von Hüfte und Schienbein entspricht einem dritten Modul (C), das geeignet ist, Hüfte und Schienbein in einer vorher eingestellten Position zu stabilisieren, so dass dem Kniegelenk Bewegungsfreiheit gelassen wird; und mindestens ein Mittel zur Korrektur des Fußgelenks und der Zehen entspricht einem vierten Modul (D), das geeignet ist, den Fuß in Bezug auf das Fußgelenk in der Frontal- und in der Saggitalebene zu stabilisieren, so dass das Fußgelenk über Bewegungsfreiheit verfügt; eine äußere Oberfläche jedes Moduls besteht aus genopptem Material, das zur Verwendung bei Befestigungen vom Typ textiler Klettverschluss geeignet ist; die drehbaren Korrekturteile (33) verbinden nacheinander das zweite, dritte und vierte Modul (B, C, D), wobei die Möglichkeit besteht, sie auch wieder von einander zu trennen; jedes drehbare Korrekturelement (33) hat die Form eines Bands aus elastischem Material, welches einen Dehnungsgrad von 5 % bis 50 % aufweist, so dass beim Patienten während dessen Fortbewegung eine Korrektur der Bewegungen stattfindet, und verfügt über eine Anzahl von Verbindungsmitteln (41), die geeignet sind, die Spannung der drehbaren Korrekturelemente (33) an jeder Stelle anzupassen, an der sie mit jedem der zweiten, dritten und vierten Module (B, C, D) verbunden sind, wobei jedes der Verbindungsmittel eine eingreifende Oberfläche hat, die geeignet ist, bei Verwendung eines textilen Klettverschlusses an der Außenfläche jedes der zweiten, dritten und vierten Module eine Verbindung herzustellen, und zwar an jeder Stelle dieser Fläche je nach Art der Erkrankung des Patienten.

2. Vorrichtung nach Anspruch 1, bei der jedes drehbare Korrekturelement (33) über ein Mittel (44) verfügt, mit dem die Länge desselben verändert werden kann.

3. Vorrichtung nach Anspruch 1, bei der die Reklinationsorthese (1) ein erstes flexibles Band (2) und ein zweites flexibles Band (2) aufweist, wobei beide Bänder räumlich in Form einer Schlaufe gebogen sind und auf diese Weise einen ersten und einen zweiten Gurt bilden, die das entsprechende Schultergelenk des Patienten umschließen und über ein Interaktionsmittel (4) verfügen, das geeignet ist, die ersten Enden (3) des ersten und des zweiten flexiblen Bands (2) miteinander zu verbinden, wobei die Möglichkeit besteht, den dazwischen liegenden Abstand anzupassen; das genannte Mittel ist auf dem Rücken des Patienten angebracht, wobei die zweiten Enden (5) des ersten und des zweiten flexiblen Bands (2) jeweils auf dem ersten und zweiten flexiblen Band (2) in der Nähe des Interaktionsmittels (4) befestigt werden.

4. Vorrichtung nach Anspruch 3, zu der ferner eine elastische Platte (13) gehört, die einen Teil des Patientenrückens im Schulterblätterbereich zwischen der Reklinationsorthese (1) und dem Rücken des Patienten bedecken soll, wobei die Außenseite dieser elastischen Platte (13) aus genopptem Material besteht, das zur Verwendung bei einer Befestigung vom Typ textiler Klettverschluss geeignet ist und zur Verbindung mit dem ersten und zweiten Band (2) der Reklinationsorthese (1) dient; die Innenseite jedes Bands hat eine eingreifende Oberfläche, die zur Verwendung bei einer Befestigung vom Typ textiler Klettverschluss geeignet ist und mit der Außenfläche der elastischen Platte (13) verbunden werden kann, deren unterer Teil über ein Verbindungsmittel (14) verfügt, das eine Verbindung mit dem zweiten Modul (B) ermöglicht.

5. Vorrichtung nach Anspruch 1, bei der das Mittel zur Korrektur des Rumpfs des Patienten ein Korsett (6) umfasst, welches eine Profilform hat, das die Wirbelsäule korrigiert, ohne dass in senkrechter Richtung eine Belastung derselben erfolgt; ein erster Teil (7) des Korsetts (6) ist geeignet, den Körper des Patienten im Lendenwirbelbereich zu umschließen, und umfasst Mittel (9) zur Befestigung dieses Teils in Querrichtung am Körper des Patienten, wohingegen ein zweiter Teil (8) des Korsetts (6) geeignet ist, den Rücken des Patienten im Bereich der Schulterblätter zu bedecken.

6. Vorrichtung nach Anspruch 4, bei der das Mittel zur Korrektur des Rumpfs des Patienten ein Korsett mit Profilform umfasst, das die Wirbelsäule korrigiert, ohne dass dabei in senkrechter Richtung eine Belastung derselben erfolgt, wobei das Korsett den Körper des Patienten im Lendenwirbelbereich umschließt und ein Mittel (12) umfasst, das eine Befestigung in Querrichtung am Körper des Patienten ermöglicht, sowie ein Verbindungsmittel (15), das geeignet ist, mit dem Verbindungsmittel (14) der elastischen Platte (13) verbunden zu werden.

7. Vorrichtung nach Anspruch 1, bei der das Mittel (16) zur Korrektur des Hüftgelenks und des Fußgelenks eine erste flexible Bandage (17) und eine zweite flexible Bandage (18) umfasst, wobei beide geeignet sind, die untere Extremität des Patienten jeweils ober- und unterhalb des Kniegelenks zu umschließen und dort befestigt zu werden, wobei die Möglichkeit besteht, den Abstand dazwischen mit Hilfe von Befestigungen (20) anzupassen, die sich an einem Ende von jeder der beiden flexiblen Bandagen (17, 18) befinden und die im Bereich der Kniekehle mit den in Längsrichtung gegenüberliegenden Kanten derselben verbunden sind.

8. Vorrichtung nach Anspruch 1, bei der das Mittel (21) zur Korrektur des Fußgelenks und der Zehen einen ersten flexiblen Gurt (22) umfasst, der den Knöchel umschließt und an einem seiner Enden ein Interaktionsmittel (24) aufweist, das geeignet ist, den flexiblen Gurt (22) mit dem Knöchel zu verbinden, wobei die Möglichkeit besteht, den Abstand dazwischen anzupassen; ein zweiter flexibler Gurt (23), der geeignet ist, den Fuß im Bereich des Längsbogens der Fußsohle zu umschließen, und dessen Enden an dem ersten flexiblen Gurt (22) auf den entgegengesetzten Knöchelseitenflächen befestigt sind; ein kreuzförmiges Zehen-/Fußsohlenelement (26) mit drei Enden (27, 28, 29) die räumlich gegeneinander gebogen sind, um den Fuß im Zehenbereich zu umschließen, wobei diese Enden durch Befestigungen (31) miteinander verbunden sind, wohingegen ein viertes Ende (30) dieses Elements frei und geeignet ist, über die gesamte Länge des Fußes unter demselben platziert zu werden; ferner sind zwei elastische Schienen (32) vorgesehen, die jeweils den ersten und zweiten Gurt (22, 23) mit dem Zehen-/Fußsohlenelement (26) im Zehenbereich verbinden, wobei die Möglichkeit besteht, den dazwischen liegenden Abstand anzupassen.

9. Vorrichtung nach Anspruch 1, zu der ferner Anpassungs- und Verbindungsmittel (42) für die Verbindung der drehbaren Korrekturelemente (33) mit einem entsprechenden Modul gehören; jedes dieser Mittel weist mindestens eine Öse (43) auf und besteht aus zwei Schichten, wovon die erste eine Außenseite aus genopptem Material hat, das zur Verwendung bei einer Befestigung vom Typ textiler Klettverschluss geeignet ist, wohingegen die äußere Oberfläche der zweiten Schicht eingreifend und zur Verwendung bei einer Befestigung vom Typ textiler Klettverschluss geeignet ist, wobei jedes dieser Verbindungsmittel, über das jedes der drehbaren Korrekturelemente (33) verfügt, eine genoppte Oberfläche aufweist, die zur Verwendung bei einer Befestigung vom Typ textiler Klettverschluss geeignet ist.

10. Vorrichtung nach Anspruch 1, zu der ferner ein fünftes Modul E gehört, das aus mindestens einem Mittel (34) zur Korrektur einer oberen Extremität gehört, das geeignet ist, Schulter und Unterarm des Patienten in einer vorher eingestellten Position zu stabilisieren, um dem Ellbogengelenk Bewegungsfreiheit zu lassen, wobei die äußere Oberfläche dieses Moduls aus genopptem Material und zur Verwendung bei einer Befestigung vom Typ textiler Klettverschluss geeignet ist; es ist wenigstens ein drehbares Korrekturelement (33) vorgesehen, das dieses fünfte Modul (E) mit dem ersten Modul (A) verbindet, wobei die Möglichkeit besteht, beide auch wieder zu trennen, und wobei dieses Element aus einem Band aus elastischem Material mit einem Dehnungsgrad von 5% bis 50% besteht und geeignet ist, die Bewegung einer oberen Extremität, während des Verlaufs dieser Bewegung, zu korrigieren; das drehbare Korrekturelement (33) ist mit einer Anzahl von Verbindungsmitteln (41) ausgestattet, die geeignet sind, die Spannung dieses Elements an der Verbindungsstelle zwischen dem ersten Modul (A) und dem fünften Modul (E) anzupassen, sowie mit einer eingreifenden Oberfläche versehen, die zur Verwendung bei einer Befestigung vom Typ textiler Klettverschluss an jeder Stelle der äußeren Oberfläche des ersten Moduls (A) und des fünften Moduls (E) geeignet ist, je nach Art der Erkrankung des Patienten.

11. Vorrichtung nach Anspruch 10, bei der das drehbare Korrekturelement (33) des Mittels (34) zur Korrektur einer oberen Extremität mit einem Mittel (44) zur Veränderung von dessen Länge ausgestattet ist.

12. Vorrichtung nach Anspruch 10 oder 11, bei der das Mittel (34) zur Korrektur einer oberen Extremität ein erstes räumlich gebogenes flexibles Band (35) und ein zweites räumlich gebogenes flexibles Band (36) umfasst, wobei beide geeignet sind, die obere Extremität jeweils über und unter dem Ellbogen zu umschließen, und dort befestigt sind, wobei die Möglichkeit besteht, den Abstand zwischen beiden mittels Befestigungen anzupassen, die an einem Ende von jedem der beiden räumlich gebogenen flexiblen Bändern (35,36) vorgesehen sind, und wobei diese flexiblen Bänder (35, 36) im subcubitalen Bereich mit ihren in Längsrichtung gegenüberliegenden Kanten miteinander verbunden sind; ferner ist ein V-förmiges Karpalelement (38) vorgesehen, das von der Ergonomie her geeignet ist, an der Handfläche des Patienten befestigt zu werden und den Daumen zu abduzieren, sowie ein Mittel (39) zur Befestigung des elastischen Karpalelements (38) an der Hand des Patienten, wobei die Möglichkeit besteht, die Spannungskraft des elastischen Karpalelements (38) anzupassen, welches über mindestens eine elastische Schiene (40), welche das flexible Karpalelement (38) und das zweite flexible Band (36) miteinander verbindet, mit dem zweiten flexiblen Band (36) verbunden ist, wobei an den Enden der Schiene (40) eine Anzahl von Verbindungsmitteln (41) vorgesehen ist, die geeignet sind, die Spannung der Schiene (40) an der Stelle anzupassen, wo sie mit dem flexiblen Karpalelement (38) und dem zweiten flexiblen Band (36) verbunden ist.

## Revendications

1. Dispositif pour traiter les patients souffrant de maladies du système nerveux central et/ou d'une blessure de leur appareil locomoteur, comprenant un dossier (1) destiné à être placé au niveau d'une région supérieure du corps du patient et adapté pour abducter les ceintures scapulaires et adducter les omoplates à la colonne vertébrale ; un moyen pour corriger la région médiane du corps du patient située dans la région lombaire de celui-ci ; au moins un moyen (16) pour corriger la hanche et le tibia avec possibilité de fixer la hanche et le tibia dans une position prédéterminée ; au moins un moyen (21) pour corriger l'articulation de la cheville et les orteils, capable de fixer le pied par rapport à l'articulation de la cheville ; une pluralité d'éléments rotatifs correcteurs (33) et une pluralité de moyens de connexion (41), dans lequel lesdits éléments du dispositif sont formés sous forme de pluralité de modules séparés (A, B, C, D) qui couvrent certaines régions du corps du patient, chacun desdits modules étant adapté pour être utilisé indépendamment ; ledit dossier (1) étant en fait un premier module (A) ; ledit moyen pour corriger la région médiane du corps du patient est essentiellement un deuxième module (B) adapté pour corriger la colonne vertébrale sans appliquer de force verticale sur celle-ci; au moins un moyen pour corriger la hanche et le tibia est un troisième module (C) adapté pour fixer la hanche et le tibia dans une position prédéterminée de façon à apporter une liberté de mouvement à l'articulation du genou ; et au moins un moyen pour corriger l'articulation de la cheville et les orteils est essentiellement un quatrième module (D) adapté pour fixer le pied par rapport à l'articulation de la cheville dans les plans frontal et sagittal afin d'apporter une liberté de mouvement dans l'articulation de la cheville ; une surface extérieure de chaque module est réalisée en une matière cotonneuse adaptée pour être utilisée dans des fixations en tissu de type Velcro ; lesdits éléments rotatifs correcteurs (33) joignent successivement lesdits deuxième, troisième et quatrième modules (B, C, D) avec possibilité de les dissocier ; chaque élément rotatif correcteur (33) est formé sous forme de bande de matière élastique ayant un pourcentage d'allongement de 5 à 50% de façon à assurer la correction du mouvement du patient durant son déplacement et comporte un nombre de moyens de connexion (41) adaptés pour régler le serrage desdits éléments rotatifs correcteurs (33) à chaque endroit de leur connexion à chacun desdits deuxième, troisième et quatrième modules (B, C, D), chacun desdits moyens de connexion ayant une surface d'engagement adaptée pour assurer une connexion au moyen d'une fixation en tissu de type Velcro au niveau de la surface extérieure de chacun des deuxième, troisième et quatrième modules à n'importe quel endroit sur ladite surface en fonction de la pathologie dont souffre le patient.

2. Dispositif selon la revendication 1, dans lequel chaque élément rotatif correcteur (33) comporte un moyen (44) pour en faire varier la longueur.

3. Dispositif selon la revendication 1, dans lequel le dossier (1) comporte une première bande souple (2) et une seconde bande souple (2), chacune d'elles étant courbée spatialement en forme de boucle de façon à former une première sangle et une seconde sangle, chacune servant à enlacer l'articulation scapulo-humérale respective du patient et comportant un moyen d'interaction (4) adapté pour interconnecter les premières extrémités (3) desdites première et seconde bandes souples (2) avec possibilité de régler l'espacement entre elles, ledit moyen étant disposé sur le dos du patient, tandis que les secondes extrémités (5) des première et seconde bandes souples (2) sont fixées sur les première et seconde bandes souples respectives (2) près dudit moyen d'interaction (4).

4. Dispositif selon la revendication 3, comprenant en outre une plaque élastique (13) pour couvrir une partie du dos du patient dans la région des omoplates entre le dossier (1) et le dos du patient ; la surface extérieure de ladite plaque élastique (13) est réalisée en une matière cotonneuse adaptée pour être utilisée dans une fixation en tissu de type Velcro pour la connexion aux première et seconde bandes (2) du dossier (1) ; le côté interne de chaque bande comporte une surface d'engagement adaptée pour être utilisée dans la fixation en tissu de type Velcro afin de connecter la surface extérieure de ladite plaque élastique (13) dont la partie inférieure comporte un moyen de connexion (14) pour la connexion au second module (B).

5. Dispositif selon la revendication 1, dans lequel le moyen pour corriger la partie médiane du corps du patient comprend un corset (6) ayant une forme profilée qui assure la correction de la colonne vertébrale sans appliquer de charge verticale sur celle-ci, une première partie (7) dudit corset (6) est adaptée pour enlacer le corps du patient dans la région lombaire et comporte un moyen (9) pour fixer transversalement ladite partie sur le corps du patient, tandis qu'une seconde partie (8) du corset (6) est adaptée pour couvrir le dos du patient dans la région des omoplates.

6. Dispositif selon la revendication 4, dans lequel le moyen pour corriger la partie médiane du corps du patient comprend un corset ayant une forme profilée pour assurer la correction de la colonne vertébrale sans appliquer de charge verticale sur celle-ci, ledit corset enlaçant le corps du patient dans la région lombaire et comportant un moyen (12) pour le fixer transversalement sur le corps du patient, et un moyen de connexion (15) adapté pour être joint au moyen de connexion (14) de ladite plaque élastique (13).

7. Dispositif selon la revendication 1, dans lequel le moyen (16) pour corriger l'articulation coxo-fémorale et l'articulation de la cheville comprend un premier bandage souple (17) et un second bandage souple (18), les deux étant adaptés pour enlacer l'extrémité inférieure du patient par-dessus et en dessous de l'articulation du genou, respectivement, et y étant fixés avec possibilité de régler l'espacement entre eux au moyen de fixations (20) prévues à une extrémité de chacun desdits bandages souples (17, 18) qui sont interconnectés dans la région poplitée avec les bords longitudinaux opposés de celle-ci.

8. Dispositif selon la revendication 1, dans lequel le moyen (21) pour corriger l'articulation de la cheville et les orteils comprend une première ceinture souple (22) qui enlace la cheville et qui comporte à l'une de ses extrémités un moyen d'interaction (24) adapté pour fixer ladite ceinture souple (22) à la cheville avec possibilité de régler la distance entre elles ; une seconde ceinture souple (23) adaptée pour enlacer le pied dans la région de l'arche plantaire longitudinale de celui-ci et ayant ses extrémités fixées à la première ceinture souple (22) sur les surfaces latérales opposées de la cheville ; un élément plantaire pour orteils en forme de croix (26) à trois extrémités (27, 28, 29) courbées spatialement les unes vers les autres pour enlacer le pied dans la région des orteils, lesdites extrémités étant interconnectées par le biais de fixations (31), tandis qu'une quatrième extrémité (30) de celui-ci est vacante et adaptée pour être disposée en dessous du pied sur toute la longueur de celui-ci; deux bretelles élastiques (32) étant également prévues pour connecter les première et seconde ceintures souples respectives (22,23) audit élément plantaire pour orteils (26) dans la région des orteils avec possibilité de régler l'espacement entre eux.

9. Dispositif selon la revendication 1, comprenant en outre un moyen de réglage-connexion (42) pour interconnecter les éléments rotatifs correcteurs (33) avec un module correspondant ; chacun desdits moyens ayant au moins une boucle de verrouillage (43) et étant composé de deux couches, la surface extérieure d'une première desdits couches étant réalisée dans une matière cotonneuse adaptée pour être utilisée dans une fixation en tissu de type Velcro, tandis que la surface extérieure de la seconde couche est une surface d'engagement adaptée pour être utilisée dans la fixation en tissu de type Velcro ; chacun desdits moyens de connexion fournis dans chacun des éléments correcteurs rotatifs (33) comporte une surface cotonneuse adaptée pour être utilisée dans une fixation en tissu de type Velcro.

10. Dispositif selon la revendication 1, comprenant en outre un cinquième module (E) formé en tant qu'au moins un moyen (34) pour corriger une extrémité supérieure, adapté pour fixer l'épaule et l'avant-bras du patient dans une position préréglée afin d'apporter une liberté de mouvement à l'articulation du coude, la surface extérieure dudit module étant réalisée en une matière cotonneuse adaptée pour être utilisée dans une fixation en tissu de type Velcro ; au moins un élément correcteur rotatif (33) étant prévu pour connecter ledit cinquième module (E) audit premier module (A) avec possibilité de les déconnecter l'un de l'autre, ledit élément étant formé sous forme de bande dans une matière élastique ayant un pourcentage d'allongement de 5 à 50% et adapté pour corriger le mouvement d'une extrémité supérieure durant son fonctionnement, ledit élément correcteur rotatif (33) étant muni d'un nombre de moyens de connexion (41) adaptés pour régler le serrage dudit élément à l'endroit de sa connexion audit premier module (A) et audit cinquième module (E), et ayant une surface d'engagement adaptée pour être utilisée dans la fixation en tissu de type Velcro à n'importe quel endroit sur les surfaces extérieures dudit premier module (A) et dudit cinquième module (E) en fonction de la pathologie dont souffre le patient.

11. Dispositif selon la revendication 10, dans lequel l'élément correcteur rotatif (33) du moyen (34) pour corriger une extrémité supérieure est muni d'un moyen (44) pour en faire varier la longueur.

12. Dispositif selon la revendication 10 ou 11, dans lequel le moyen (34) pour corriger une extrémité supérieure comprend une première bande souple courbée spatialement (35) et une seconde bande souple courbée spatialement (36), les deux étant adaptées pour enlacer l'extrémité supérieure par-dessus et en dessous de l'articulation du coude, respectivement, et étant fixées à celle-ci avec possibilité de régler la distance entre elles au moyen de fixations fournies à une extrémité de chacune desdites bandes souples courbées spatialement (35, 36), lesdites bandes souples (35,36) étant interconnectées dans la région sous-cubitale par leurs bords longitudinaux opposés ; un élément carpien souple en forme de V (38) étant aussi fourni, adapté ergonomiquement pour être fixé à la paume du patient et pour abducter le pouce, et un moyen (39) pour fixer ledit élément carpien souple (38) à la main du patient avec possibilité de régler la force de serrage dudit élément carpien souple (38) qui est connecté à la seconde bande souple (36) par le biais d'au moins une bretelle élastique (40) qui interconnecte ledit élément carpien souple (38) à ladite seconde bande souple (36), un nombre de moyens de connexion (41) étant prévu aux extrémités de ladite bretelle (40), adaptés pour régler le serrage de ladite bretelle (40) à l'endroit de sa connexion audit élément carpien souple (38) et à ladite seconde bande souple (36).
